Europäisches Patentamt

European Patent Office

Office européen des brevets

(19)

(11) Publication number: **0 081 370**
**A1**

(12) **EUROPEAN PATENT APPLICATION**

(21) Application number: **82306488.6**

(22) Date of filing: **06.12.82**

(51) Int. Cl.³: **A 61 K 31/415**
**A 61 K 9/00**

(30) Priority: **07.12.81 US 327834**

(43) Date of publication of application:
**15.06.83 Bulletin 83/24**

(84) Designated Contracting States:
**BE CH DE FR GB IT LI LU NL SE**

(71) Applicant: **SYNTEX (U.S.A.) INC.**
**3401 Hillview Avenue P.O. Box 10850**
**Palo Alto California 94303(US)**

(72) Inventor: **Vickery, Brian H.**
**10678 Farallone Drive**
**Cupertino California 95014(US)**

(74) Representative: **Armitage, Ian Michael et al,**
**MEWBURN ELLIS & CO. 2/3 Cursitor Street**
**London EC4A 1BQ(GB)**

(54) **Disposable intravaginal contraceptive devices releasing 1-substituted imidazoles.**

(57) Devices, of suitable form and size for insertion into the vagina of a female mammal, composed of a silastic polymer which has, incorporated in it, a spermicide that is 1-[2-(2,4-dichlorobenzyloxy)-n-octyl]imidazole, or a pharmaceutically acceptable acid addition salt thereof, and which release effective amounts of said spermicide while in place in the vaginal cavity, are useful as contraceptives.

-1-

# DISPOSABLE INTRAVAGINAL CONTRACEPTIVE
## DEVICES RELEASING 1-SUBSTITUTED IMIDAZOLES

The present invention relates to methods and devices for contraception which depend upon spermicidal activity of a 1-substituted imidazole applicable to the female mammal intravaginally. More specifically, the present invention concerns intravaginal inserts, such as diaphragms, rings, or cervical cups which contain, within the matrix of the silastic polymer material of which they are composed, a specific 1-substituted imidazole spermicide, and which devices are capable of releasing an effective amount of said spermicide at a controlled rate in situ.

Present methods of contraception suffer from a variety of well known defects. Systemically active drugs which alter the ovulatory cycle of the female, whether taken orally, or inserted in the uterus or vagina for slow release, cause a substantial number of side effects ranging from discomfort and menstrual irregularities to life threatening alterations in metabolism. Mechanical barriers, such as condoms and diaphragms, in their present form, are not ideally effective, and, more importantly, do not have maximum user acceptance because of the necessity of their being employed in the same psychological time frame as intercourse. Spermicidal preparations designed for intravaginal application are even less effective and less aesthetically acceptable than the aforesaid mechanical barrier devices. The goal of a contraceptive which is a) effective b) confined in its metabolic effects to contraception itself, and c) can be employed at a time far psychologically removed from the sex act, has not been achieved in the prior art.

Intravaginal devices for release of contraceptive materials are well known, and many designs have been described: for example, a vaginal contraceptive tampon is described in international application published under PCT 80/00008 (claiming priority from U.S. application 888,578); a vaginal medicator in U.S. 3,885,564; a vaginal ring in U.S. 3,545,439; a diaphragm in U.S. 2,087,610, and a cervical cap in U.S. 2,836,177.

Devices for the release of a progestational agent as the active ingredient have also been described: (WHO Special Programme of Research, Development and Research Training in Human Reproduction, Geneva, Switzerland: Journal of Steroid Biochemistry 11 (1B) 461 (1979); Burton, F.G., et al, Contraception 17 (3) 221 (1978); Gordon, N.R., et al, Saf. Health Plast. Nat'l. Tech. Conf. Soc. Plast Engr. 1977, 109. Victor, A., et al, Contraception, 16 (2) 125 (1977), Zanartu, et al, Steroids 21 (3) 325 (1973); British Patent Application 2,616,064. However, the results have been unsatisfactory because of continuing problems with unwanted metabolic alterations and because of the difficulty of assuring proper release of the steroid (WHO study, supra). As systemic contraceptives, progestational compounds are subject to causing such side effects regardless of the mode of administration.

Release of spermicides, because the effect is not systemic to the female host, is an inherently more desirable option. Devices which release spermicides of the surfactant type have also been described: Stone, et al, Am. J. Obstet. and Gynecol. 133 (6): 635 (1979), U.S. Patent 4,031,202, British Patent 1,329,619, European Patents 9-417 and 9-518. However, surfactant spermicides are effective only at relatively high concentrations, and devices which remain in the vaginal cavity are incapable of releasing effective amounts over long periods. Furthermore, surfactants are not effective within the cervical mucus. Hence, effectiveness is impaired because the critical sperm pathway through the mucus to effect conception remains unimpaired.

The present invention utilizes a specific spermicide 1-[2-(2,4-dichlorobenzyloxy)-n-octyl]imidazole incorporated into a specific type of polymeric material, namely a silastic polymer. It has been found that, with this choice of spermicide and polymeric material, intravaginal devices made therefrom are capable of releasing sufficient quantities into a suitable location in the vagina to maintain effectiveness. The devices and method of this invention, thus permit provision for contraception directed specifically against spermatozoa at times psychologically displaced from the time of intercourse.

It should be noted for the sake of completeness that the use of this imidazole as a spermicide is suggested in U.S. Patent No. 4,247,552, but in this U.S. Patent there is no disclosure of the incorporation of the compound into a silastic polymer to form an intravaginal device in the manner of this invention nor of the desirable properties obtained thereby.

The present invention concerns a device of suitable configuration for vaginal insertion and long term retention in the female mammal, which is composed of an inert flexible silastic polymeric material wherein the polymerization is effected in the presence of a spermicide that is 1-[2-(2,4-dichlorobenzyloxy)-n-octyl]imidazole, or a pharmaceutically acceptable acid addition salt thereof and wherein the resulting polymer is permeable to the release of said spermicide.

This device is useful for effecting contraception. Accordingly, another aspect of the present invention concerns a method of contraception using said device.

Definitions:

As used herein:

"Spermicide" or "spermicidal" includes those agents which kill sperm, and also those which otherwise immobilize or render sperm ineffective in fertilization;

"Inert" means material which does not dissolve in, or become absorbed by, the fluids or structures of the vaginal cavity, and maintains its structural and configurational integrity in the vaginal environment;

"Pharmaceutically acceptable acid addition salt" means those salts which retain the spermicidal properties of the free base and which are neither biologically or otherwise undesirable, formed with, for example, inorganic acids such as hydrochloric acid, hydrobromic acid, sulfuric acid, nitric acid or phosphoric acid; or inorganic acids such as acetic acid, propionic acid, glycolic acid, lactic acid, pyruvic acid, oxalic acid, malonic acid, succinic acid, malic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid, and the like;

Article of Manufacture

### A. Configuration

The device of the invention herein is intended to be insertable into, and retained in, the vagina for extended time periods, easily removable, comfortable, and non-interfering with intercourse. It is designed to remain in the vagina between menstrual periods, and to be disposable at the end of each use; to be self-inserted (unlike intrauterine devices which must be inserted by a physician) and to be of non-critical dimensions, so that it need not be fitted by a physician (as is the case with diaphragms).

### Description of the Drawing

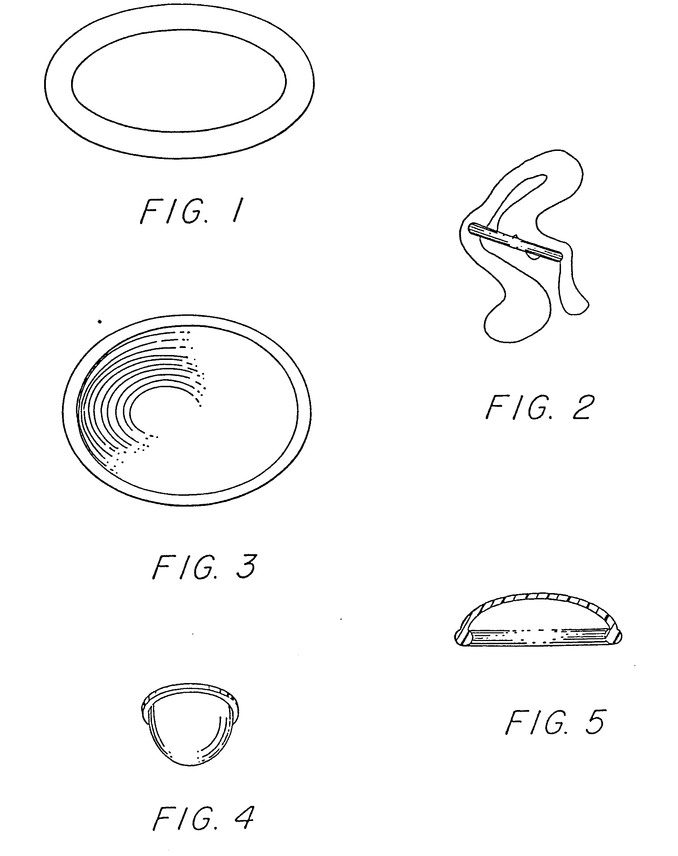

An exemplary embodiment of the configuration of the invention is a ring such as that shown in Fig. 1, which is 70-80 mm across and 4-10 mm in diameter, and flexible so as to be retained between the rear wall of the vagina and the upper edge of the pubic bone, as shown in Fig. 2. The insertion and removal of such a device may be done by hand by the user.

Another embodiment of the configuration of the invention, exemplified in Fig. 3, is a diaphragm, which is essentially similar to the above ring in dimensions, properties and usability, except that the ring supports a membrane of thin flexible material. As the effectiveness of the device herein-described does not depend on its attributes as a mechanical barrier, this embodiment of the invention does not require fitting by a physician, as is the case with diaphragms in general.

Another preferred embodiment, exemplified in Fig. 4, is a cervical cap. As is the case with diaphragm, the efficacy of this embodiment of the device does not depend on its properties as a barrier. Therefore, exact fit is not critical, and user insertion is feasible. The cap is held in place by suction, and may be removed by hand.

Figure 5 shows a cross-section of a "diaphragm" which comprises a single molded piece of polymeric material which holds the spermicide in the matrix of the polymer.

The preceeding drawing depicts suitable embodiments of the device which are intended to be exemplary, and not limiting. Any device which consists as a whole, or in part, of a silastic polymeric material capable of incorporating the spermicide described herein into the matrix of the polymer, and which will be retained in the vagina is encompassed by the invention.

B. Composition of the Matrix

The matrix which constitutes the structure and configuration of the device is composed of inert, flexible silastic polymeric material which has been polymerized in the presence of, and as to include within inself, an effective amount of the spermicide. Additionally, if desired, polymerization may be carried out so as to incorporate other therapeutically active or contraceptive materials, as well as the spermicide, such as antifungal agents, hormones, pH control agents, and the like.

Preparation and regulation of the physical properties of said silastic polymeric materials are well known to those skilled in the art. Standard methods of preparation and property control for these polymers may for example, be found in the Modern Plastics Encyclopedia, published by McGraw-Hill, New York, New York, U.S.A.

Suitable silastic polymers are employed, such as those described in U.S. Patent 3,269,996. In a still more preferred embodiment, the silastic is a silicone elastomer such as Dow-Corning 382 silicone elastomer polymerized in the presence of a catalyst.

In a typical preparation, the spermicide 0.05%-10% by weight, preferably 0.5%-5% is stirred into a paste containing the silicone elastomer and a suitable catalyst as essentially the balance of the mixture, and optionally, other therapeutically active or contraceptive ingredients. The mixture is placed into a mold suitable for the configuration such as those described above, and allowed to set. The proportion of catalyst to monomer will depend on the degree of flexibility desired, but will range from 10%-30% of the entire mixture, preferably about 20% for the uses described herein.

Optional additional therapeutically active or contraceptive ingredients may include, without limitation, bacteria controlling agents, such as, for example, penicillins, tetracyclines, or streptomycins or their pharmaceutically acceptable salts; antiinflammatory agents, such as, for example, cortisone or prednisolone or their salts; estrogens or other hormones, such as, for example, estradiol, prostaglandins; or progesterone; or physiologically safe acids such as tartaric, citric or boric, which serve to lower the ambient pH and thus create a hostile environment for sperm cells.

C. Spermicide

The effectiveness of the device and method of contraception described herein depends upon the use, as the spermicide, of the compound 1-[2-(2,4-dichloro-benzyloxy)-n-octyl]imidazole, or a pharmaceutically acceptable acid addition salt thereof. This compound has been found to be an effective spermicide at low concentrations, and to be capable of entering the cervical mucus.

The spermicide has a chiral carbon atom, and the spermicide component as disclosed herein may be one of the two enantiomeric forms, or some mixture thereof.

Method of Contraception

The method of the present invention comprises effecting contraception using the article of manufacture described hereinabove. In this aspect of the invention, the device described herein is inserted and retained in the vaginal cavity in such a manner that an effective concentration of spermicide is maintained during the lifetime of the sperm introduced during intercourse.

Two considerations determine the preferred method of the invention. One is, of course, that the times of insertion and removal of the device must be such that an effective concentration of the spermicide is maintained during the time of viability of sperm. The other is the psychological aspect - it is preferred that insertion be at a time removed from intercourse, and that removal be at will. Devices currently available are limited in accommodating these two aspects simultaneously - i.e., in order to maintain an effective level of spermicide, insertion must be made relatively close to the time of intercourse (within the general psychological time frame) and removal must be postponed for an unduly long period.

In the method of the present invention, the effectiveness level is designed to be maintained so as to permit insertion more than eight hours before intercourse (although times closer can be used, too, of course) and so that removal can be done essentially at the preference of the user.

Accordingly, in the preferred method, insertion is made at a time close enough to the time of intercourse to insure an effective level of spermicide, and removal is made at a time which does not destroy the level of effectiveness while it is still needed.

The device of the present invention may be inserted, if so desired, at least as much as 8 hours before intercourse and may be removed, if so desired, immediately thereafter. It is contemplated that the device would be removed during menstruation, and that ordinarily its total residence time in the vagina would not be greater than one month. The device may be used repeatedly until the internal concentration of spermicide is decreased below an effective level.

The following examples are intended to illustrate, but not to limit, the invention:

### EXAMPLE I
Spermicide Releasing Matrix

A preparation of the following composition was made:

1-[2-(2,4-dichlorobenzyloxy)-n-octyl]imidazole

| | |
|---|---|
| oxalate: (spermicide) | 0.1 g |
| Dow Corning 382 Silicone elastomer | 2.4 g |
| Catalyst M | 20 drops |
| Dow Corning 360 Medical Silicone Fluid | 4 drops |

The preparation was stirred to a paste and placed in a mold of rod shape for 4 hours at room temperature.

From the average rod weight of 80.3 mg, a 3.21 mg of spermicide per rod was calculated.

(Silicone 382 elastomer is an opaque viscous elastomer base composed of polydimethylsiloxane and silica filler. Catalyst M is composed of stannous octoate. 360 Medical Silicone Fluid is a clear, colourless, polydimethylsiloxane liquid. All are available from Dow Corning Corporation, Midland, Michigan, 48640, United States of America.)

EXAMPLE 2

Three rods prepared as in Example 1 were placed in 200 ml normal saline in a dissolution basket and incubated at 37°C. Samples of the normal saline were withdrawn at 2 hour intervals and assayed for spermicide.

Satisfactory release rates per rod per hour of spermicide were found for 8 hour and 24 hour intervals.

EXAMPLE 3

Six white, soft, elastic cervical rings were prepared, each of weight 0.5 g., outer diameter 2.3 cm, inner diameter 1.5 cm.

The rings were made from:

| | |
|---|---|
| 1-(2-(2,4-dichlorobenzyloxy)-N-octyl)imidazole malate | 0.01 g |
| Dow Corning Silicone 382 elastomer | 0.6 g |
| Catalyst M | 5 drops |

The procedure was as follows. The spermicide was mixed well with the elastomer, and to the resultant mixture was added the catalyst with thorough mixing with a spatula. The resultant paste was carefully transferred to molds by small increments to avoid trapping of air. The molds were then allowed to stand overnight at room temperature for the elastomer to cure, whereafter smooth white rings were obtained from the molds.

EXAMPLE 4

A ring prepared as in Example 3 was placed intravaginally in a female stumptailed Macaque (maintained in an estrogen-dominated physiological state by subcutaneously implanted silastic capsules of estradiol - cervical mucus in such an animal continuously possesses the mucus characteristics only seen around the time of ovulation in normal Macaques).

The ring was maintained in place for six days, and then removed.   24 hours after ring removal, the female was mated, and postcoital analysis revealed the following:

SPERM RECOVERIES

Vaginal Fluid

| Sperm Conc. No. x $10^6$ | Percent Motility | Forward Progression (0-4) |
| --- | --- | --- |
| 178 | 90 | 4 plus |

Cervical Mucus

| Sperm Conc. (per grid) | Percent Motility | Forward Progression (0-4) |
| --- | --- | --- |
| 360 | 10 | 0 |

These results are indicative of the contraceptive effects achievable in the cervical mucus with devices according to the invention.

CLAIMS:

1.    A contraceptive device which releases spermicide during intravaginal placement in a female mammal comprising

a)    an inert, flexible silastic polymeric material formed into a configuration suitable for vaginal insertion and long term retention in the vagina and permeable to the release of said spermicide;

b)    an effective amount of a spermicide that is 1-[2-(2,4-dichlorobenzyloxy)-n-octyl]imidazole or a pharmaceutically acceptable acid addition salt thereof, included within the matrix of said polymeric material.

2.    A device of Claim 1 wherein the configuration is that of a ring of suitable size to fit into the vagina.

3.    A device of Claim 1 wherein the configuration is that of a diaphragm of suitable size to fit into the vagina.

4.    A device of Claim 1 wherein the configuration is that of a cervical cap.

5.    A device of any one of the preceding claims wherein the polymeric material is formed from silicone elastomer.

6.    A device of any one of the preceding claims wherein said polymeric material is formed by polymerization carried out in the presence of an effective amount of said spermicide.

7. A method of contraception which comprises insertion of a device of any one of the preceding claims into the vagina of a female mammal.

8. The method of claim 7 wherein insertion is made at a time psychologically displaced from the time of intercourse.

9. The device of claim 1, for use as a contraceptive.

10. A process for the preparation of the device of claim 1, which process comprises polymerising the silastic material in the presence of the spermicide.

1/1

FIG. 1

FIG. 2

FIG. 3

FIG. 4

FIG. 5

## DOCUMENTS CONSIDERED TO BE RELEVANT

EP 82306488.6

| Category | Citation of document with indication, where appropriate, of relevant passages | Relevant to claim | CLASSIFICATION OF THE APPLICATION (Int. Cl. ³) |
|---|---|---|---|
| A | GB - A - 2 066 067 (SQUIBB & SONS INC.) <br> * Abstract; page 1; page 4, left column * | 1-4, 7-10 | A 61 K 31/415 <br> A 61 K 9/00 |
| A | US - A - 4 247 552 (HALLESY et al.) <br> * Columns 1-4; claims * | 1-4, 7-10 | |
| A | US - A - 4 277 475 (VICKERY) <br> * Columns 1-4; claims * | 1-4, 7-10 | |
| A | GB - A - 1 531 693 (RICHARDSON-MERRELL INC.) <br> * Page 5, Depot-Implant, lines 28-35 * | 5,6 | |
| A | US - A - 4 264 578 (BURCK et al.) <br> * Columns 5-7 * | 5,6 | TECHNICAL FIELDS SEARCHED (Int. Cl. ³) <br><br> A 61 K 31/00 <br> A 61 K 9/00 <br> A 61 F 5/00 |
| A | US - A - 3 929 984 (ZAFFARONI) <br> * Abstract; examples 6,7,11 * | 5,6 | |
| A | AU - A - 46 535/72 (MOYER) <br> * Claims 9,10 * | 5,6 | |
| A | FR - A1 - 2 287 917 (SCHERING AKTIENGESELLSCHAFT) <br> * Claims * | 5,6 | |

The present search report has been drawn up for all claims

| Place of search | Date of completion of the search | Examiner |
|---|---|---|
| VIENNA | 16-02-1983 | EBERLE |

| | DOCUMENTS CONSIDERED TO BE RELEVANT | | CLASSIFICATION OF THE APPLICATION (Int. Cl. 3) |
|---|---|---|---|
| Category | Citation of document with indication, where appropriate, of relevant passages | Relevant to claim | |
| A | GB - A - 1 522 846 (SEARLE & CO) <br><br> * Totality, especially example 1 * <br><br> -- | 5,6 | |
| A | GB - A - 1 412 970 (SCHERING AKTIENGESELLSCHAFT) <br><br> * Claim 14 ff * <br><br> ----- | 5,6 | |
| | | | **TECHNICAL FIELDS SEARCHED (Int. Cl. 3)** |

EPO Form 1503.2  08.78